# EUROPEAN PATENT APPLICATION

(11) **EP 4 806 349 A1**
(43) Date of publication of application: **16.09.2026**
(21) Application number: 26163283.0
(22) Date of filing: 09.03.2026
(51) Int. Cl.: A61B 5/145

(54) **IMPLANTATION DEVICE FOR ANALYTE SENSOR**

(30) Priority: 14.03.2025 CN 202510305867
(71) Applicant: Jiangsu Yuwell POCTech Biotechnology Co., Ltd., Zhenjiang, Jiangsu 212300 (CN); Jiangsu Yuekai Biotechnology Co., Ltd., Nanjing, Jiangsu 210018 (CN); Zhejiang Poctech Co., Ltd., Huzhou, Zhejiang 313001 (CN)
(72) Inventor: ZHANG, Yanan, Nanjing, 210018 (CN); ZHU, Jiandong, Nanjing, 210018 (CN); WANG, Liang, Nanjing, 210018 (CN); MCCANLESS, Jonathan, Nanjing, 210018 (CN)
(74) Representative: Lorenz Seidler Gossel Part. mbB

(57) **Abstract**

This application discloses an implantation device for an analyte sensor, including: an outer housing, provided with an accommodating cavity; a monitoring unit, arranged inside the accommodating cavity and including a first module and a second module, where the first module and the second module are spaced apart; and a driving unit, arranged inside the accommodating cavity and configured to drive the monitoring unit to move along a first direction, where in a process in which the driving unit drives the monitoring unit to move along the first direction, at least one of the first module and the second module moves toward the other of the first module and the second module, to complete assembly between the first module and the second module. The first module and the second module of the monitoring unit are arranged independently of each other, and therefore, can be sterilized in two different sterilization manners. Alternatively, a separation barrier may be installed between the first module and the second module to implement local sterilization, thereby simplifying a sterilization process. The monitoring unit is already assembled with the outer housing before delivery. After obtaining a product, a user does not need to perform an additional assembly step, so that operation steps are reduced.

## Description

### TECHNICAL FIELD

This application relates to the field of medical equipment technologies, and specifically, to an implantation device for an analyte sensor.

### BACKGROUND

Detecting various analytes of an individual *in vivo* is crucial for monitoring the health of the individual, and a deviation from a normal analytic level may usually indicate an underlying physiological condition, for example, a metabolic status or a disease. Performing *in vitro* analyte monitoring regularly by using extracted body fluids is sufficient to observe physiological conditions of many individuals. However, due to a limited quantity of measured values in *in vitro* analyte monitoring, there is a possibility of missing an optimal treatment window due to a loss of crucial measurement data causing irreversible harm to a patient. In addition, for an individual with severe analyte disorders and/or rapidly fluctuating analyte levels, body fluids need to be extracted frequently for monitoring, which is inconvenient and painful for the patient.

In many cases, a subcutaneous, interstitial, or skin analyte sensor can provide sufficient measurement accuracy while minimizing user discomfort. Continuous analyte monitoring using an *in vivo* implantable analyte monitoring sensor is an ideal monitoring method. Generally, an analyte monitoring sensor is implanted into the body of an individual by using an implantation device. The sensor reacts with body fluids of the implantee to generate an electrical signal. After converting the electrical signal into data capable of representing an analyte concentration, a processing unit transmits the data to a display device for display, thereby implementing continuously monitoring of the analyte concentration.

Usually, a sensor is inserted into the body of a user by using an implantation device. A puncture needle inside the implantation device is engaged with the sensor and brings the sensor under the skin of the user, after the sensor is implanted in place, the puncture needle is removed from the body of the user while the sensor remains in the body of the user. The existing products have technical problems of complex sterilization processes and many user operation steps while having an increased probability of a sensor implantation failure due to an improper user operation.

### SUMMARY

This application provides an implantation device for an analyte sensor capable of simplifying a production process and reducing user operation steps, to improve user experience. The implantation device includes:
an outer housing, provided with an accommodating cavity; a monitoring unit, arranged inside the accommodating cavity and including a first module and a second module, where the first module and the second module are spaced apart; and
a driving unit, arranged inside the accommodating cavity and configured to drive the monitoring unit to move along a first direction, where in a process in which the driving unit drives the monitoring unit to move along the first direction, at least one of the first module and the second module moves in a direction toward the other of the first module and the second module, to complete assembly between the first module and the second module.

In a possible implementation, the direction toward the other of the first module and the second module has a displacement perpendicular to the first direction.

In a possible implementation, projections of the first module and the second module along the first direction do not overlap.

In a possible implementation, the first module and the second module are spaced apart along a second direction, and at least one of the first module and the second module move along the second direction to complete assembly between the first module and the second module, where the second direction is perpendicular to the first direction.

In a possible implementation, the first module includes a data transmission unit, the second module includes a data obtaining unit, where the first module moves toward the second module, to complete assembly between the data transmission unit and the data obtaining unit.

In a possible implementation, the monitoring unit has an initial state and an assembled state, where in the initial state, the first module and the second module are spaced apart, in the assembled state, assembly between the first module and the second module is completed; and in the initial state, positions at which the first module and the second module are located in the first direction have a same height.

In a possible implementation, the driving unit includes a driving member, the driving member is provided with a driving slope, and in a process in which the first module and/or the second module moves along the first direction, the driving slope drives the first module and/or the second module to deviate toward each other.

In a possible implementation, the driving unit includes a driving member and a transmission member, where the transmission member is arranged between the driving member and the monitoring unit, and the driving member drives the transmission member to move, to push the first module and/or the second module to move, to implement assembly between the first module and the second module.

In a possible implementation, the implantation device further includes a guiding member, where the guiding member is arranged inside the accommodating cavity, the guiding member and/or the transmission member is provided with a guiding slope that gradually inclines from the first direction toward a central axis of the accommodating cavity, and in a process in which the driving member moves along the first direction, the guiding slope drives the transmission member to move, to push the first module and/or the second module to deviate toward each other.

In a possible implementation, the transmission member includes a guiding portion, and the driving member is provided with a guiding groove, where the guiding groove extends along the second direction, and the second direction is perpendicular to the first direction.

In a possible implementation, the implantation device further includes an unlocking member, where the driving unit includes a locking portion, the outer housing includes a stopping portion, the locking portion cooperates with the stopping portion to restrict movement of the driving unit, the unlocking member is arranged at one end of the outer housing along the first direction and is movable along the first direction to apply a force to the stopping portion or the locking portion to disengage the stopping portion and the locking portion.

In a possible implementation, the implantation device further includes a puncture unit, arranged inside the accommodating cavity, and including a puncture needle, where the second module is provided with a through hole, and the puncture unit is fixed to the second module and the puncture needle passes through the through hole.

In a possible implementation, the implantation device further includes a sealing member, where the second module includes a sensor, the sealing member abuts against a bottom surface of the second module to form a sealed cavity, and the sensor and the puncture needle are at least partially located inside the sealed cavity.

In a possible implementation, the second module includes a sensor and a battery, and when assembly between the second module and the first module is completed, the battery is electrically connected to the first module.

In a possible implementation, a first electrical connecting portion is arranged on a side of the first module facing the second module, and a second electrical connecting portion is arranged on a side of the second module facing the first module, where the first electrical connecting portion is coupled to the second electrical connecting portion.

In a possible implementation, the implantation device further includes a shielding member, arranged at one end of the outer housing, where the shielding member and the outer housing jointly configure the accommodating cavity into a sealed space.

In a possible implementation, the implantation device includes a bottom housing, where the bottom housing is detachably connected to the outer housing to cover the accommodating cavity.

Because the foregoing technical solutions are used, beneficial effects obtained by this application are as follows:
In this application, the first module and the second module of the monitoring unit are fixed and spaced apart inside the accommodating cavity, and are arranged independently of each other, and therefore, the two modules can be sterilized in two different sterilization manners. Alternatively, a separation barrier may be installed between the two to implement local sterilization, so that complexity of the sterilization process is reduced while effectively prevent irradiation sterilization from causing damage to electronic units, thereby simplifying a production process. In addition, the monitoring unit is already assembled with the outer housing before delivery. After obtaining a product, a user does not need to perform an additional assembly step, so that steps that the user needs to perform are reduced, thereby lowering use difficulty and improving use convenience. Further, after an implantation action is triggered, assembly between the first module and the second module is completed under driving of the driving unit, which also does not require the user to perform an additional operation, thereby greatly simplifying operation steps performed by the user in a use process, lowering difficulty in operating a product, reducing learning costs of the user, and improving user experience.

### BRIEF DESCRIPTION OF THE DRAWINGS

The accompanying drawings described herein are used to provide a further understanding of this application, and form a part of this application. Exemplary embodiments of this application and description thereof are used to explain this application, and do not constitute any inappropriate limitation to this application. In the accompanying drawings:
FIG. 1 is a schematic structural diagram of an implantation device in an implementation of this application;
FIG. 2 is a cross-sectional view of an implantation device in an implementation of this application;
FIG. 3 is a schematic structural diagram of an implantation device in an implementation of this application, where an outer housing is not shown;
FIG. 4 is a schematic structural diagram of a partial structure of an implantation device in an implementation of this application;
FIG. 5 is a schematic structural diagram of a partial structure of an implantation device in another implementation of this application;
FIG. 6 is a schematic structural diagram of a monitoring unit in an implementation of this application;
FIG. 7 is a schematic structural diagram of a monitoring unit in another implementation of this application; and
FIG. 8 is a cross-sectional view of an implantation device in an implementation of this application.

In the figures:
1. outer housing; 11. accommodating cavity; 12. limiting groove; 13. implantation port; 14. stopping portion;
2. unlocking member; 21. unlocking slope;
3. bottom housing; 31. protruding rib;
4. driving unit; 41. booster spring; 42. driving member; 421. guiding groove; 422. force-transmitting portion; 43. guiding channel; 44. transmission member; 441. pushing portion; 442. force-bearing portion; 443. guiding portion; 45. limiting portion; 46. locking portion; 47. elastic rib;
5. puncture unit; 51. tension spring; 52. needle hub; 53. puncture needle;
6. guiding member; 61. guiding slope; 62. avoidance groove; 63. extending portion;
7. monitoring unit; 71. first module; 711. positioning protrusion; 712. first electrical connecting portion; 72. second module; 721. through hole; 722. positioning groove; 723. second electrical connecting portion; 724. sensor; 73. adhesive attachment;
8. sealing member; 81. sealed cavity.

### DETAILED DESCRIPTION

To more clearly explain the overall concept of this application, detailed descriptions are provided below by using examples with reference to the accompanying drawings.

In the following descriptions, many specific details are provided to give a full understanding of this application. However, this application may alternatively be implemented in other manners different from those described herein. Therefore, the protection scope of this application is not limited to the specific embodiments disclosed below.

In addition, in the description of this application, it should be understood that orientation or position relationships indicated by the terms such as "top", "bottom", "inner", "outer", "axial", "radial", "circumferential", and the like are based on orientation or position relationships shown in the accompanying drawings, and are used only for ease and brevity of description of this application, rather than indicating or implying that the mentioned device or element needs to have a particular orientation or needs to be constructed and operated in a particular orientation. Therefore, such terms should not be construed as a limitation to this application.

In this application, unless explicitly specified or limited otherwise, the terms "mounted", "connected", "connection", and "fixed" should be understood broadly, for example, which may be fixed connections, detachable connections or integral connections; or may be a mechanical connection, an electrical connection, or communication; or may be a direct connection, an indirect connection through an intermediate, or internal communication between two elements or an interaction relationship between two elements. A person of ordinary skill in the art may understand the specific meanings of the foregoing terms in the present invention according to specific situations.

In this application, unless explicitly specified or limited otherwise, a first characteristic "on" or "under" a second characteristic may be the first characteristic in direct contact with the second characteristic, or the first characteristic in indirect contact with the second characteristic by using an intermediate medium. In the descriptions of this specification, descriptions using reference terms such as "implementation", "embodiment", "an embodiment", "example", or "specific example" mean that specific characteristics, structures, materials, or features described with reference to the embodiment or example are included in at least one embodiment or example of this application. In this specification, schematic representations of the above terms are not necessarily directed to the same embodiments or examples. Moreover, the specific features, structures, materials, or characteristics described may be combined in any one or more embodiments or examples in a suitable manner.

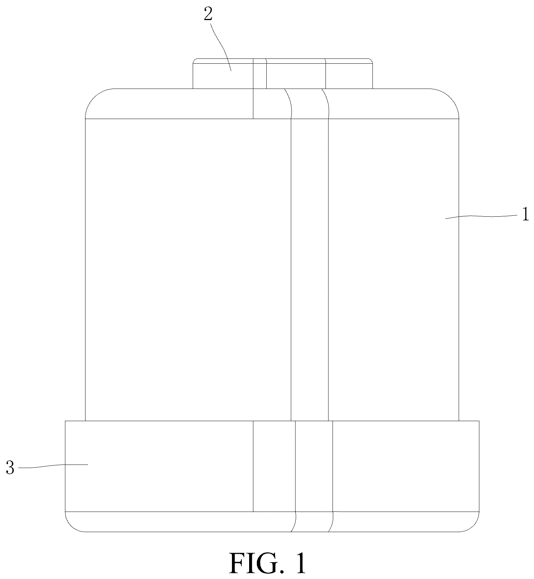

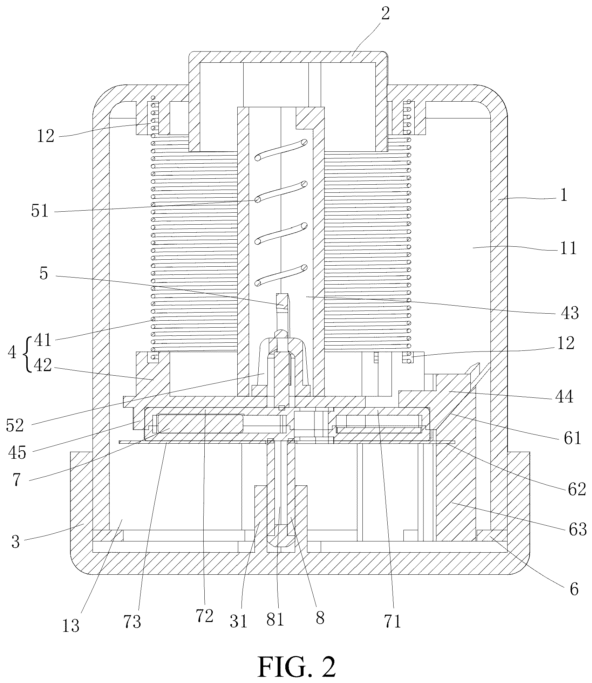

As shown in FIG. 1 and FIG. 2, an implantation device for an analyte sensor 724 includes: an outer housing 1, provided with an accommodating cavity 11; a monitoring unit 7, arranged inside the accommodating cavity 11 and including a first module 71 and a second module 72, where the first module 71 and the second module 72 are spaced apart; and a driving unit 4, arranged inside the accommodating cavity 11 and configured to drive the monitoring unit 7 to move along a first direction, where in a process in which the driving unit 4 drives the monitoring unit 7 to move along the first direction, at least one of the first module 71 and the second module 72 moves toward the other of the first module 71 and the second module 72, to complete assembly between the first module 71 and the second module 72.

Preferably, the first direction is an axial direction of the outer housing 1.

In this application, the first module 71 and the second module 72 of the monitoring unit 7 are fixed and spaced apart inside the accommodating cavity 11, and are arranged independently of each other, and therefore, the two modules can be sterilized in two different sterilization manners. Alternatively, a separation barrier may be installed between the two to implement local sterilization, so that complexity of the sterilization process is reduced while effectively prevent irradiation sterilization from causing damage to electronic units, thereby simplifying a production process.

For example, in an embodiment, the first module 71 and the second module 72 are spaced apart, and an irradiation separator is arranged between the first module 71 and the second module 72, so that cavities in which the first module 71 and the second module 72 are located are separated from each other, to enable local sterilization. When the sensor is sterilized through irradiation sterilization, electronic units are prevented from being damaged by irradiation. Preferably, the separator should be made of a material that can block irradiation and gases.

In addition, the monitoring unit 7 is already assembled with the outer housing 1 before delivery. After obtaining a product, a user does not need to perform an additional assembly step, so that steps that the user needs to perform are reduced, thereby lowering use difficulty and improving use convenience. Further, after an implantation action is triggered, assembly between the first module 71 and the second module 72 is completed under driving of the driving unit 4, which also does not require the user to perform an additional operation, thereby greatly simplifying operation steps performed by the user in a use process, lowering difficulty in operating a product, reducing learning costs of the user, and improving user experience.

After an implantation action is triggered, assembly between the first module 71 and the second module 72 can be automatically completed under driving of the driving unit 4, which also does not require the user to perform an additional operation and requires only one trigger operation. In a process in which the driving unit 4 moves along the first direction, assembly between the first module 71 and the second module 72 needs to be completed first, and then, the entire monitoring unit 7 moves along the first direction to implant the sensor 724 under the skin of the host, and an adhesive attachment 73 at a bottom surface of the monitoring unit 7 is adhered and fixed to the skin of the user, for the user to wear. In this way, once one trigger operation is implemented, a plurality of steps are automatically completed. Operation steps performed by the user in an implantation process are greatly simplified, which lowers difficulty in operating the product, and reduces learning costs of the user. In addition, duration of the entire implantation stage is also shortened, which helps reduce fear of the user in waiting for the implantation.

For example, the sensor 724 may be arranged in the first module 71, and a transmitter may be arranged in the second module 72. After the two modules are respectively sterilized in different manners (for example, the first module 71 is sterilized through irradiation sterilization, and the second module 72 is sterilized through gas sterilization), the first module 71 and the second module 72 are separately fixed inside the accommodating cavity 11, and then subsequent packaging and delivery are performed.

As shown in FIG. 2, an implantation port 13 is provided at one end of the outer housing 1 along the first direction. During use, the user butts the one end provided with the implantation port 13 against the skin surface, to cause the skin surface to cover the implantation port 13, and then triggers the implantation device to complete implantation of the sensor 724.

In the related art, in some monitoring units, a sensor module and a transmitter module that are stacked vertically, that is, projections of the sensor module and the transmitter module in a vertical direction overlap, resulting in an increased entire height (thickness) of a housing of the monitoring unit and a notable protrusion in appearance. Consequently, after the monitoring unit is adhered and fixed to the skin surface of a user, the monitoring unit is prone to friction with clothing of the user, especially on parts such as the abdomen and upper arms. In addition, a surface area of an adhesive attachment on a bottom surface of the monitoring unit is small, which directly affects a contact area between the adhesive attachment and the skin surface. As a result, adhesion stability of the adhesive attachment is poor, and the adhesive attachment cannot conform well to the curvature of the skin surface, and consequently, is prone to detachment. In addition, the monitoring unit has concentrated pressure distribution on the skin surface and large local pressure, and easily causes discomfort when being worn for a long time.

A manner of assembling the first module 71 and the second module 72 is not limited in this application. In an embodiment, as shown in FIG. 2 to FIG. 5, in a process of assembling the first module 71 and the second module 72, a position of the second module 72 keeps unchanged, and the first module 71 moves toward the second module 71, to complete assembly between the first module 71 and the second module 72. Alternatively, the first module 71 keeps stationary, and the second module 72 moves toward to the first module 72, to complete assembly. Still alternatively, the first module 71 and the second module 72 move toward each other, to complete assembly.

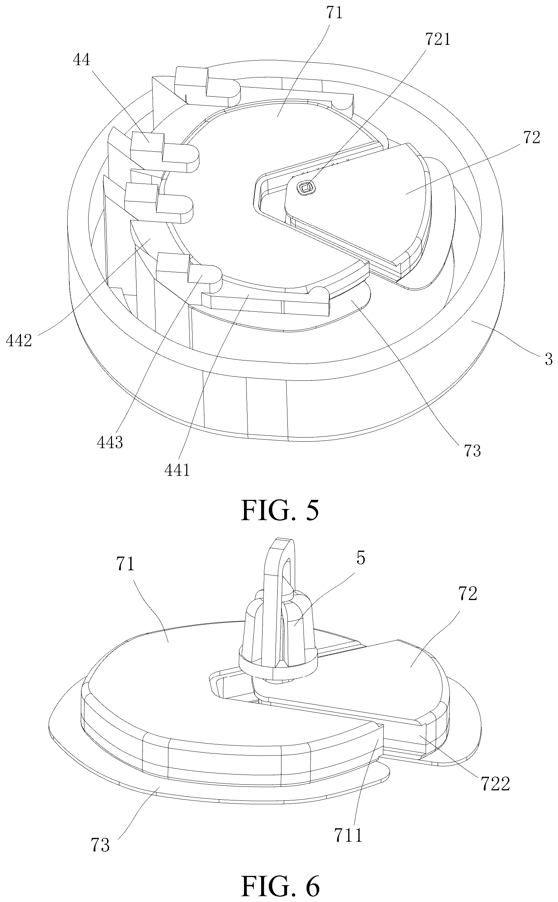

In a preferred implementation of this application, as shown in FIG. 5 and FIG. 6, projections of the first module 71 and the second module 72 along the first direction do not overlap.

It should be noted that in this application, the monitoring unit 7 has an initial state in which the first module 71 and the second module 72 are spaced part and an assembled state in which assembly of the first module 71 and the second module 72 is completed. Regardless of in the initial state or in the assembled state, projections of the first module 71 and the second module 72 along the first direction do not overlap. The first module 71 and the second module 72 in FIG. 5 and FIG. 6 are in an unassembled state.

After the first module 71 and the second module 72 are assembled, the monitoring unit 7 has an overall height directly and effectively reduced, thereby reducing a probability of friction with clothing after being put on. In addition, a surface area of the bottom surface of the monitoring unit 7 is also increased, to optimize distribution of an adhesive on a surface of the adhesive attachment 73 to resist a shearing force, so that anti-twisting performance of the monitoring unit 7 is improved, and the monitoring unit 7 can be adhered to the skin surface more firmly, and better conform to the curvature of the skin surface, thereby improving stability of adhesion between the monitoring unit 7 and the skin surface.

In addition, the monitoring unit 7 distributes pressure on the skin surface more evenly, can reduce local pressure, and is especially suitable for wearing for a long time, thereby improving wearing comfort.

In addition, electronic components in modules generate some heat during operation, and there is no overlap between two modules, which is more beneficial to heat dissipation, thereby avoiding an excessive local temperature rise of the monitoring unit 7 from causing discomfort to the user.

It should be noted that a direction of assembling the first module 71 and the second module 72 is not limited in this application. In a preferred implementation, as shown in FIG. 2 to FIG. 7, the first module 71 and the second module 72 are spaced apart along a second direction, and at least one of the first module 71 and the second module 72 move along the second direction to complete assembly between the first module 71 and the second module 72, where the second direction is perpendicular to the first direction.

Preferably, the second direction is a direction perpendicular to an axis of the outer housing 1.

Assembly the first module 71 and the second module 72 is completed in a direction perpendicular to the first direction, which can avoid large collisions and squeezing forces between the two modules in the first direction under a driving force of the driving unit 4 along the first direction from causing large vibration or shaking, and avoid a risk that a violent collision causes an unstable structural connection and an unstable electrical connection. In addition, after assembly the first module 71 and the second module 72 is completed along the second direction, which helps to achieve that projections of the two along the first direction do not overlap.

In a preferred embodiment of this implementation, as shown in FIG. 2, the monitoring unit 7 has an initial state and an assembled state, where in the initial state, the first module 71 and the second module 72 are spaced apart, in the assembled state, assembly between the first module 71 and the second module 72 is completed; and in the initial state, positions at which the first module 71 and the second module 72 are located in the first direction have a same height.

In an initial state, the first module 71 and the second module 72 are arranged at a same horizontal height, so that the two modules are more easily and reliably assembled along the second direction. After the implantation action is triggered, assembly between the first module 71 and the second module 72 is completed in a direction perpendicular to the axis of the outer housing 1, and then, the first module 71 and the second module 72 move together along the first direction to perform the implantation action.

It should be noted that assembly between the first module 71 and/or the second module 72 can be completed by moving along a single direction, that is, the second direction, as described in the foregoing implementation. Alternatively, assembly may be completed by moving along another direction, provided that there is a displacement in the second direction. For example, in another implementation, during assembly, the first module 71 and/or the second module 72 have displacements in both the first direction and the second direction, that is, move along an inclined direction to move toward each other to complete assembly.

In a specific example, when both the first module 71 and the second module 72 move along the first direction, the first module 71 and the second module 72 also move toward a central axis of the outer housing 1 along the second direction, so that assembly between the two is completed along an inclined moving track.

In another specific example, in the initial state, positions at which the first module 71 and the second module 72 are located have different heights in the first direction, that is, the first module 71 and the second module 72 are at different horizontal heights. For example, the second module 72 is higher than the first module 71, during assembly, while moving along the first direction, the second module 72 also move toward the central axis of the outer housing 1 along the second direction, to complete assembly with the first module 71 below.

Certainly, in another implementation, assembly between the first module 71 and the second module 72 may alternatively be completed along the first direction. This is not limited herein.

Preferably, the first module 71 includes a data transmission unit, the second module 72 includes a data obtaining unit, where the first module 71 moves toward the second module 72, to complete assembly between the data transmission unit and the data obtaining unit.

It may be understood that, the data obtaining unit is the sensor 724, the data transmission unit of the first module 71 is suitable for irradiation sterilization, and the data obtaining unit of the second module 72 is suitable for gas sterilization. After the two modules are respectively sterilized in different manners, the first module 71 and the second module 72 are separately fixed inside the accommodating cavity 11, and then subsequent packaging and delivery are performed. After the implantation action is triggered, assembly of the two is completed to form the completed monitoring unit 7, so that the monitoring unit 7 can work normally.

In addition, a puncture unit 5 is fixed to the second module 72, and the second module 72 keeps stationary during assembly, which can ensure stability of the position of the puncture unit 5.

In an embodiment, the first module 71 further includes a data processing unit. The data processing unit may be arranged inside the first module 71, or may be arranged outside the first module 71, for example, a mobile device end such as a mobile phone. This is not limited herein.

It should be noted that a driving manner of the driving unit 4 is not limited in this application. The driving unit 4 may be manually driven, that is, the user manually drives the driving unit 4 to move along the first direction. Preferably, as shown in FIG. 2, FIG. 3, and FIG. 8, the driving unit 4 includes a booster spring 41 and a driving member 42. The booster spring 41 abuts between an inner wall of the outer housing 1 and the driving member 42. The power of the driving member 42 is provided by the booster spring 41. In the initial state, the driving member 42 is locked inside the outer housing 1. In this case, the booster spring 41 is in a compressed state. After the user triggers unlocking, the booster spring 41 pushes the driving member 42 under an elastic force of the booster spring 41, so that the driving member 42 moves along the first direction to perform the implantation action. In this way, not only automatic implantation is implemented, but also a moving speed of the driving member 42 can be better controlled by using driving of the booster spring 41, so that the driving member 42 tends to have a constant speed and a more stable moving direction, to avoid jamming during movement, thereby reducing shaking to alleviate pain of the user during implantation.

Further, as shown in FIG. 2, an inner wall of the outer housing 1 and/or the driving member 42 is provided with a limiting groove 12, and an end of a booster spring 41 is arranged in the limiting groove 12. The limiting groove 12 can limit the end of the booster spring 41, so that the booster spring 41 always deforms along an axial direction of the booster spring 41, and provides a stable acting force along the first direction for the driving member 42. Specifically, as shown in FIG. 2, limiting grooves 12 are provided on both the driving member 42 and the outer housing 1, so that both ends of the booster spring 41 are located in the limiting grooves 12.

A manner of driving the first module 71 and the second module 72 to complete assembly is not limited in this application. In an implementation, the driving unit 4 includes a driving member 42, the driving member 42 is provided with a driving slope, and in a process in which the first module 71 and/or the second module 72 moves along the first direction, the driving slope drives the first module 71 and/or the second module 72 to deviate toward each other.

It should be noted that according to different relative positions of the driving member 42 and the monitoring unit 7, the driving slope have different inclination directions. For example, in an embodiment, the driving member 42 is located above the monitoring unit 7, a driving slope is provided on a lower side of the driving member 42, and the driving slope gradually inclines from the first direction away from a central axis of the accommodating cavity 11.

In a process of moving along the first direction, the driving member 42 is in contact with the first module 71 and/or the second module 72 through the driving slope, thereby generating an acting force perpendicular to the first direction from an acting force along the first direction through the driving slope, thereby pushing the first module 71 and/or the second module 72 to get close to each other to complete assembly. Preferably, the driving slope is in contact with an outer side wall of the first module 71 and/or the second module 72, to perform pushing in a direction toward the central axis of the outer housing 1.

Certainly, in another embodiment, the first module 71 and/or the second module 72 may be provided with a slope structure that gradually inclines from the first direction away from the central axis of the accommodating cavity 11. Through contact between the slope structure and the driving member 42, the driving member 42 can also be enabled to generate a transverse pushing force for the first module 71 and/or the second module 72.

In another embodiment, the driving member 42 includes a pushing structure located at a transverse side of the monitoring unit 7, the driving slope is arranged on the pushing structure, and the driving slope gradually inclines from the first direction toward the central axis of the accommodating cavity 11, to push the first module 71 and/or the second module 72 toward the central axis of the outer housing 1 during movement along the first direction. The pushing structure may be a pushing device such as a spring. When the user triggers the implantation device, the pushing structure is also triggered, to form a pushing force for the first module 71 and/or the second module 72 to drive assembly of the two.

In the foregoing embodiments, the driving member 42 is in direct contact with the monitoring unit 7 to directly drive the monitoring unit 7, which can improve transmission efficiency, and improve movement smoothness and stability of the first module 71 and the second module 72.

In another implementation of this application, as shown in FIG. 2 to FIG. 5, the driving unit 4 includes a driving member 42 and a transmission member 44, where the transmission member 44 is arranged between the driving member 42 and the monitoring unit 7, and the driving member 42 drives the transmission member 44 to move, to push the first module 71 and/or the second module 72 to move, to implement assembly between the first module 71 and the second module 72.

In this implementation, the driving member 42 drives the first module 71 and/or the second module 72 through the transmission member 44, to implement indirect driving. Specifically, in an embodiment, a slope structure may be arranged between the driving member 42 and the transmission member 44, so that the two are in contact through the slope, thereby forming a pushing force perpendicular to the first direction for the transmission member 44 from a driving force of the driving member 42 in the first direction, to cause the transmission member 44 to push the first module 71 and/or the second module 72 to move to complete assembly.

Preferably, as shown in FIG. 2 to FIG. 5, the implantation device further includes a guiding member 6, where the guiding member 6 is arranged inside the accommodating cavity 11, the guiding member 6 and/or the transmission member 44 is provided with a guiding slope 61 that gradually inclines from the first direction toward a central axis of the accommodating cavity 11, and in a process in which the driving member 42 moves along the first direction, the guiding slope 61 drives the transmission member 44 to move, to push the first module 71 and/or the second module 72 to deviate toward each other.

Specifically, the driving member 42 can drive the transmission member 44 to move together. In a process in which the transmission member 44 moves along the first direction, the transmission member 44 is guided by the guiding slope 61 to move along a direction perpendicular to the first direction, thereby pushing the first module 71 and/or the second module 72 to move.

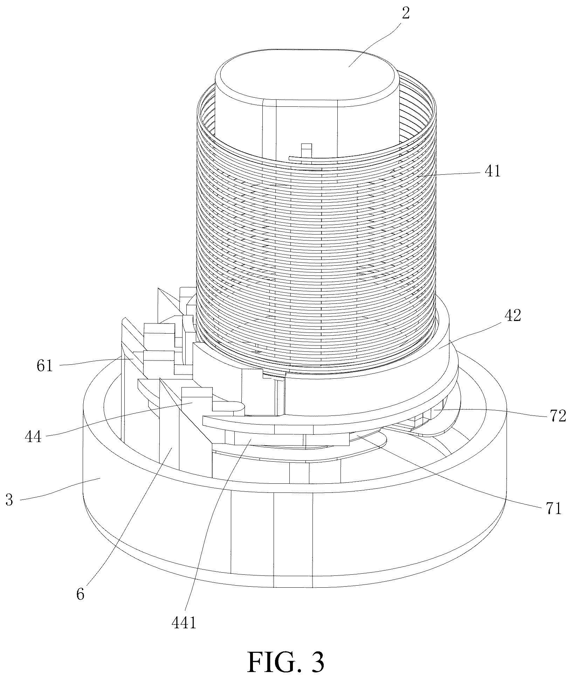

Preferably, as shown in FIG. 3 to FIG. 5, the transmission member 44 is also provided with a cooperating slope cooperating with the guiding slope 61.

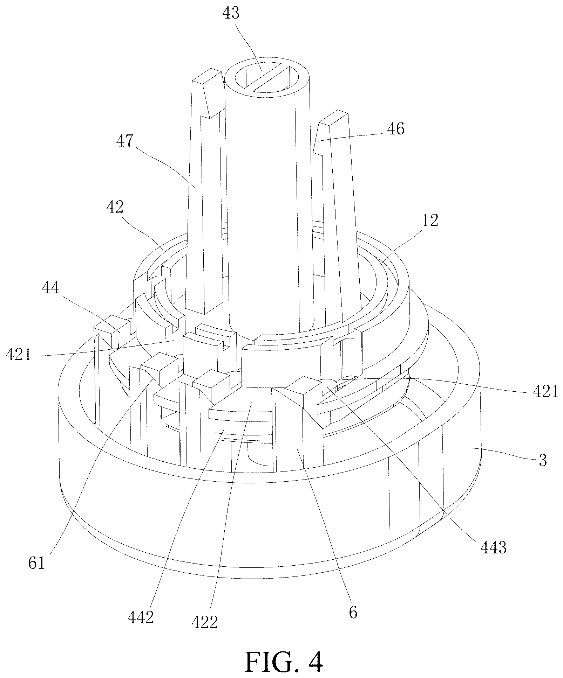

Further, as shown in FIG. 3 and FIG. 4, the transmission member 44 includes a guiding portion 443, and the driving member 42 is provided with a guiding groove 421, where the guiding groove 421 extends along the second direction, and the second direction is perpendicular to the first direction.

The guiding groove 421 forms a guide for movement of the transmission member 44 along the second direction, to cause the transmission member 44 to move along the guiding groove 421 toward the second direction in a process of moving along the first direction.

Specifically, as shown in FIG. 3 to FIG. 5, the transmission member 44 further includes a force-bearing portion 442 and a pushing portion 441. The driving member 42 has a force-transmitting portion 422, and the force-bearing portion 442 and the force-transmitting portion 422 overlap each other in the first direction, so that in a process in which the driving member 42 moves along the first direction, the force-transmitting portion 422 pushes the force-bearing portion 442 to move the transmission member 44 together in the first direction. The pushing portion 441 is located on a side of an outer periphery of the first module 71 and/or the second module 72, and at least partially surrounds the first module 71 and/or the second module 72, to increase a contact area between the transmission member 44 and the first module 71 and/or the second module 72, and apply a stable pushing force to the transmission member 44.

In an implementation in which both the first module 71 and the second module 72 can move to complete assembly, the pushing portions 441 are arranged opposite to each other on two sides of the monitoring unit 7 along a radial direction of the outer housing 1. As shown in FIG. 2 and FIG. 3, in an implementation, during assembly of the monitoring unit 7, the second module 72 keeps stationary, the first module 71 moves toward the second module 72, and the second module 72 and the transmission member 44 are arranged opposite to each other on two sides of the first module 71. As shown in FIG. 2, the driving member 42 has a limiting portion 45, the limiting portion 45 abuts against an outer side wall of the first module 71 for limiting, and the limiting portion 45 is arranged opposite to the transmission member 44.

Preferably, as shown in FIG. 4 and FIG. 5, the transmission member 44 has an arc-shaped structure, and a plurality of guiding portions 443 are arranged at intervals in an extending direction. The force-bearing portion 442 is formed between two adjacent guiding portions 443. The driving member 42 is provided with guiding grooves 421 corresponding to the guiding portions 443, and a plurality of force-transmitting portions 422 is in a one-to-one correspondence with the force-bearing portions 442.

Preferably, as shown in FIG. 2, the guiding member 6 is fixed to one end (one end provided with an implantation port 13) of the outer housing 1 along the first direction, and has an extending portion 63 extending along a direction opposite to the first direction. The extending portion 63 is located at an outer side of the monitoring unit 7 in a radial direction of the outer housing 1. A guiding slope 61 is provided on the extending portion 63. An adhesive attachment 73 is provided at a bottom surface of the monitoring unit 7. The extending portion 63 has an avoidance groove 62 for avoiding the adhesive attachment 73, to prevent adhesive attachment 73 from wrinkling and affecting adhesion stability.

Preferably, adhesive attachments 73 are arranged on bottom surfaces of both the first module 71 and the second module 72.

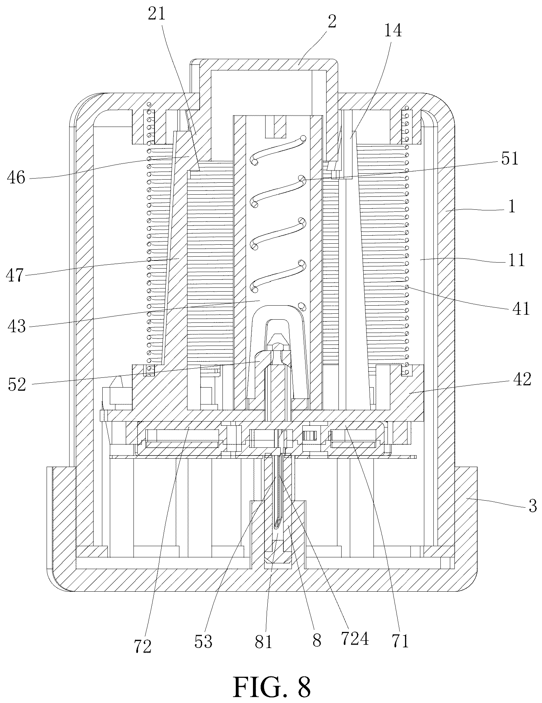

In a preferable implementation of this application, as shown in FIG. 8, the implantation device further includes an unlocking member 2, where the driving unit 4 includes a locking portion 46, the outer housing 1 includes a stopping portion 14, the locking portion 46 cooperates with the stopping portion 14 to restrict movement of the driving unit 4, the unlocking member 2 is arranged at one end of the outer housing 1 along the first direction and is movable along the first direction to apply a force to the stopping portion 14 or the locking portion 46 to disengage the stopping portion 14 and the locking portion 46.

Preferably, an opening is provided at one end of the outer housing 1, and the unlocking member 2 passes through the opening, so that the unlocking member 2 is partially located outside the outer housing 1 and partially located inside the outer housing 1. Preferably, the unlocking member 2 is a button. A cross-sectional area of the unlocking member 2 is smaller than a cross-sectional area of the accommodating cavity 11, so that the end of the outer housing 1 further has a wall surface surrounding an outer periphery of the unlocking member 2, thereby achieving miniaturized design of the unlocking member 2. Certainly, an end of the outer housing 1 may be completely opened to form an opening, so that the unlocking member 2 can be directly guided by an inner peripheral surface of the accommodating cavity 11 to move in the first direction.

Specifically, as shown in FIG. 8, the stopping portion 14 is a snap-fitting groove extending in the first direction at an end of the outer housing 1. The locking portion 46 is a snap-fitting hook arranged on the driving unit 4. The snap-fitting hook extends into the snap-fitting groove to form locking. The unlocking member 2 is provided with an unlocking slope 21. When the unlocking member 2 moves in the first direction, the unlocking slope 21 abuts against the snap-fitting hook, to cause the snap-fitting hook to disengage from the snap-fitting groove, thereby completing unlocking of the driving unit 4.

Preferably, as shown in FIG. 4 and FIG. 8, an elastic rib 47 is further arranged on the driving unit 4. The elastic rib 47 is fixed at one end and free at the other end. The locking portion 46 is arranged at the free end of the elastic rib 47.

Certainly, the stopping portion 14 may also be set as a snap-fitting hook, and the locking portion 46 is correspondingly a snap-fitting groove, or both the stopping portion 14 and the locking portion 46 are snap-fitting hooks. This is not limited herein.

Preferably, as shown in FIG. 2, FIG. 6, and FIG. 8, the implantation device further includes a puncture unit 5, arranged inside the accommodating cavity 11, and including a puncture needle 53, where the second module 72 is provided with a through hole 721, and the puncture unit 5 is fixed to the second module 72 and the puncture needle 53 passes through the through hole 721.

The puncture unit 5 is assembled with the second module 72 into one piece in advance, so that a stylus of the sensor 724 of the second module 72 can be accommodated inside the puncture needle 53 in advance, thereby omitting a step of assembling the puncture needle 53 and the stylus of the sensor 724 during implantation, and improving the implantation efficiency. In addition, the puncture unit 5 is fixed to the second module 72. Therefore, during assembly between the first module 71 and the second module 72, if the second module 72 moves, the puncture unit 5 can also synchronously move with the second module 72, to prevent a displacement in the first direction due to relative movement between the puncture unit 5 and the second module 72 from causing misalignment between the puncture needle 53 and the through hole and an implantation failure.

Preferably, the driving unit 4 is provided with a guiding channel 43, and the puncture unit 5 further includes a needle hub 52. The needle hub 52 is located in the guiding channel 43, to guide movement of the puncture unit 5. Specifically, a tension spring 51 is provided inside the guiding channel 43. One end of the tension spring 51 is fixed to the puncture unit 5. When the puncture unit 5 moves to an end of its stroke along the first direction, the stylus of the sensor 724 has been implanted under the skin of the user. The tension spring 51 is in a stretched state, and the puncture unit 5 is triggered to perform unlocking, thereby moving in a direction opposite to the first direction to perform a needle withdrawal action.

In a preferable implementation, as shown in FIG. 2 and FIG. 8, the implantation device further includes a sealing member 8, where the second module 72 includes a sensor 724, the sealing member 8 abuts against a bottom surface of the second module 72 to form a sealed cavity 81, and the sensor 724 and the puncture needle 53 are at least partially located inside the sealed cavity 81.

The sealing member 8 cooperates with the bottom surface of the monitoring unit 7 to form the sealed cavity 81, and a stylus of the sensor 724 and the puncture needle 53 are at least partially located in the sealed cavity 81, so that before the sealing member 8 is disassembled, a needle tip of the puncture needle 53 and the stylus of the sensor 724 are both located in a sterile environment isolated from the outside.

The sealing member 8 may abut against a bottom surface of the second module 72, or may partially abut against a bottom surface of the first module 71 and partially abut against a bottom surface of the second module 72. Preferably, the bottom surface of the monitoring unit 7 has an adhesive attachment 73, and the adhesive attachment 73 is provided with an avoidance region excluding an adhesive, for abutting against the sealing member 8.

In a preferable implementation, the implantation device includes a bottom housing 3, where the bottom housing 3 is detachably fixed to an end of the outer housing 1 to cover the accommodating cavity 11. In an embodiment, the bottom housing 3 has a protruding rib 31 protruding toward the monitoring unit 7, and the protruding rib 31 abuts against the bottom surface of the monitoring unit 7 and surrounds the puncture needle 53, to form the sealing member 8. In another embodiment, the sealing member 8 and the bottom housing 3 are structures independent of each other. Preferably, as shown in FIG. 2, the bottom housing 3 is provided with a limiting wall. The limiting wall surrounds an outer periphery of the sealing member 8, to limit the sealing member 8.

In a preferred implementation of this application, the second module 72 includes a sensor 724 and a battery, and when assembly between the second module 71 and the first module 72 is completed, the battery is electrically connected to the first module 71.

Before assembly between the second module 71 and the first module 72 is completed, the battery and the second module 72 are also in a separated state, and electronic components on the battery and the first module 71 are not electrically connected. Therefore, a case in which a battery is discharged before being used by a user can be reduced, warehousing time is also increased, electricity storage of the battery is ensured, and after implantation into the user, the battery a longer battery life, improving the user experience.

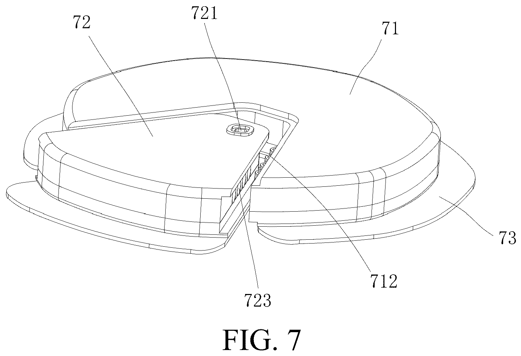

Further, as shown in FIG. 7, a first electrical connecting portion 712 is arranged on a side of the first module 71 facing the second module 72, and a second electrical connecting portion 723 is arranged on a side of the second module 72 facing the first module 71, where the first electrical connecting portion 712 is coupled to the second electrical connecting portion 723. While a structural connection between the first module 71 and the second module 72 is completed, a coupling between the first electrical connecting portion 712 and the second electrical connecting portion 723 is also completed, to implement an electrical connection.

Specifically, as shown in FIG. 7, the first electrical connecting portion 712 is arranged on a side wall of the first module 71, the second electrical connecting portion 723 is arranged on a side wall of the second module 72, and the two are coupled along the second direction. The second direction is perpendicular to the first direction. In this way, a direction of the electrical coupling is consistent with a direction of the structural connection, and the structural connection and the electrical connection between the first module 71 and the second module 72 can be synchronously completed. Preferably, the first module 71 and the second module 72 can also move along the second direction to complete assembly, thereby completing the structural connection and the electrical connection during movement without an additional operation.

Specifically, as shown in FIG. 6 and FIG. 7, one side of the first module 71 is provided with an installation notch matching a shape of the second module 72, and the second module 72 is installed in the installation notch in a direction perpendicular to the first direction. One of an inner wall of the installation notch and/or an outer wall of the second module 72 is provided with a positioning groove 722, and the other of the inner wall of the installation notch and/or the outer wall of the second module 72 is provided with a positioning protrusion 711 matching the positioning groove 722, to implement a positioning function for installation of the two.

In an implementation, the implantation device further includes a shielding member, arranged at one end of the outer housing 1, where the shielding member and the outer housing 1 jointly configure the accommodating cavity 11 into a sealed space. In this way, after components are installed inside the accommodating cavity 11, the outer housing 1 is entirely sterilized. After the sterilization is completed, the accommodating cavity 11 is sealed by using the shielding member, to form a sterile environment inside.

The shielding member may be an end cap, a sealing film fixed to one end of the outer housing 1, or the like. This is not limited herein.

Preferably, as shown in FIG. 2 and FIG. 8, the implantation device includes a bottom housing 3, where the bottom housing 3 is detachably connected to the outer housing 1 to cover the accommodating cavity 11. After covering the accommodating cavity 11, the bottom housing 3 may cooperate with the outer housing 1 to seal the accommodating cavity 11. In this case, the bottom housing 3 is a shielding member. Certainly, the bottom housing 3 may alternatively only shield the accommodating cavity 11 without sealing the accommodating cavity 11. This is not limited herein.

Before use, the user removes the bottom housing 3 from the outer housing 1, so that one end of the outer housing 1 forms an implantation port 13 in communication with the accommodating cavity 11, then abuts the end of the outer housing 1 against the skin surface, to cause the skin surface to cover the implantation port 13, and then, performs a trigger operation on the implantation device.

A manner of disassembling the bottom housing 3 is not limited in this implementation. For example, the bottom housing 3 may be separated from the outer housing 1 by moving the bottom housing 3 along the first direction, or the bottom housing 3 may be connected to the outer housing 1 in a threaded manner, and the bottom housing 3 is separated from the outer housing 1 by rotating the bottom housing 3. This is not limited herein.

The parts that are not described in this application may be implemented by using or by referring to the related art.

Embodiments in this specification are all described in a progressive manner, for same or similar parts in embodiments, reference may be made to these embodiments, and each embodiment focuses on a difference from other embodiments.

The foregoing descriptions are merely embodiments of this application and are not intended to limit this application. For a person skilled in the art, various modifications and variations can be made to this application. Any modification, equivalent replacement, or improvement made without departing from the spirit and principle of this application shall fall within the scope of the claims of this application.

## Claims

1. An implantation device for an analyte sensor, comprising:
an outer housing, provided with an accommodating cavity;
a monitoring unit, arranged inside the accommodating cavity and comprising a first module and a second module, wherein the first module and the second module are spaced apart; and
a driving unit, arranged inside the accommodating cavity and configured to drive the monitoring unit to move along a first direction, wherein
in a process in which the driving unit drives the monitoring unit to move along the first direction, at least one of the first module and the second module moves in a direction toward the other of the first module and the second module, to complete assembly between the first module and the second module.

2. The implantation device according to claim 1, wherein the direction toward the other of the first module and the second module has a displacement perpendicular to the first direction.

3. The implantation device according to claim 1 or claim 2, wherein projections of the first module and the second module along the first direction do not overlap.

4. The implantation device according to claim 1 or claim 2, wherein the first module and the second module are spaced apart along a second direction, and at least one of the first module and the second module move along the second direction to complete assembly between the first module and the second module, wherein the second direction is perpendicular to the first direction.

5. The implantation device according to any one of claims 1 to 4, wherein the first module comprises a data transmission unit, the second module comprises a data obtaining unit, wherein the first module moves toward the second module, to complete assembly between the data transmission unit and the data obtaining unit.

6. The implantation device according to claim 4, wherein the monitoring unit has an initial state and an assembled state, wherein in the initial state, the first module and the second module are spaced apart, in the assembled state, assembly between the first module and the second module is completed; and in the initial state, positions at which the first module and the second module are located in the first direction have a same height.

7. The implantation device according to any one of claims 1 - 6, wherein the driving unit comprises a driving member, the driving member is provided with a driving slope, and in a process in which the first module and/or the second module moves along the first direction, the driving slope drives the first module and/or the second module to deviate toward each other.

8. The implantation device according to any one of claim 1 - 7, wherein the driving unit comprises a driving member and a transmission member, wherein the transmission member is arranged between the driving member and the monitoring unit, and the driving member drives the transmission member to move, to push the first module and/or the second module to move, to implement assembly between the first module and the second module.

9. The implantation device according to claim 8, wherein the implantation device further comprises a guiding member, wherein the guiding member is arranged inside the accommodating cavity, the guiding member and/or the transmission member is provided with a guiding slope that gradually inclines from the first direction toward a central axis of the accommodating cavity, and in a process in which the driving member moves along the first direction, the guiding slope drives the transmission member to move, to push the first module and/or the second module to deviate toward each other.

10. The implantation device according to claim 8, wherein the transmission member comprises a guiding portion, and the driving member is provided with a guiding groove, wherein the guiding groove extends along the second direction, and the second direction is perpendicular to the first direction.

11. The implantation device according to any one of claims 1 - 10, wherein the implantation device further comprises an unlocking member, wherein the driving unit comprises a locking portion, the outer housing comprises a stopping portion, the locking portion cooperates with the stopping portion to restrict movement of the driving unit, the unlocking member is arranged at one end of the outer housing along the first direction and is movable along the first direction to apply a force to the stopping portion or the locking portion to disengage the stopping portion and the locking portion.

12. The implantation device according to any one of claims 1 - 10, wherein the implantation device further comprises a puncture unit, arranged inside the accommodating cavity, and comprising a puncture needle, wherein the second module is provided with a through hole, and the puncture unit is fixed to the second module and the puncture needle passes through the through hole; optionally the implantation device further comprises a sealing member, wherein the second module comprises a sensor, the sealing member abuts against a bottom surface of the second module to form a sealed cavity, and the sensor and the puncture needle are at least partially located inside the sealed cavity.

13. The implantation device according to any one of claims 1 - 12, wherein the second module comprises a sensor and a battery, and when assembly between the second module and the first module is completed, the battery is electrically connected to the first module; optionally a first electrical connecting portion is arranged on a side of the first module facing the second module, and a second electrical connecting portion is arranged on a side of the second module facing the first module, wherein the first electrical connecting portion is coupled to the second electrical connecting portion.

14. The implantation device according to any one of claims 1 - 13, wherein the implantation device further comprises a shielding member, arranged at one end of the outer housing, wherein the shielding member and the outer housing jointly configure the accommodating cavity into a sealed space.

15. The implantation device according to any one of claims 1 - 14, wherein the implantation device comprises a bottom housing, wherein the bottom housing is detachably connected to the outer housing to cover the accommodating cavity.
